# EUROPÄISCHE PATENTSCHRIFT

(11) **EP 1 287 787 B1**
(45) Veröffentlichungstag und Bekanntmachung des Hinweises auf die Patenterteilung: **28.10.2015**
(21) Anmeldenummer: 01120083.9
(22) Anmeldetag: 21.08.2001
(51) Int. Cl.: A61B 17/72, A61B 17/80, A61B 17/68

(54) **Implantat zur gegenseitigen Fixierung zweier Knochenfragmente**
Implant for the fixation of bone fractures
Implant pour fixation des fractures osseuses

(43) Veröffentlichungstag der Anmeldung: 05.03.2003
(73) Patentinhaber: WALDEMAR LINK GmbH & Co. KG, 22339 Hamburg (DE); Stoffella, Rudolf, Dr., 2340 Mödling (AT)
(72) Erfinder: Stoffella Rudolf Dr., 2340 Mödling (AT)
(74) Vertreter: Glawe, Delfs, Moll

(56) Entgegenhaltungen:
- WO-A-00/06036
- DE-U- 29 709 289
- GB-A- 2 266 246
- US-A- 5 976 141

## Beschreibung

Die Erfindung betrifft ein Implantat zur gegenseitigen Fixierung zweier Knochenfragmente, insbesondere zur Fixierung eines in der Achse korrigierten Köpfchens eines Mittelfußknochens, z.B. Hallux valgus, das eine Spange mit zwei Schenkeln aufweist, die an ihren einen Enden miteinander verbunden sind und in diesem Bereich eine Öse mit einer Öffnung bilden, durch die eine in einem der beiden Knochenfragmente einschraubbare Schraube hindurchgeführt ist, und die mit ihren anderen, freien Enden in das andere Knochenfragment einführbar sind.

Osteotomien zur Behandlung des Hallux valgus sind bereits seit Jahrzehnten bekannt und haben das Ziel, die Achse des Metatarsale I wieder funktionell zu rekonstruieren. Hierbei ist es erforderlich, nach der Osteotomie die beiden Knochenfragmente in ihrer korrigierten Stellung zu fixieren, um eine interfragmentäre Beweglichkeit zu verhindern und eine sichere knöcherne Heilung ohne Dislokation zu ermöglichen.

Es ist bereits bekannt, zur Fixierung der Osteotomie Platten zu verwenden, die über mehrere Schrauben an der Kortikalen befestigt werden, um ein Ausknicken der Knochenfragmente zu verhindern. Hierbei ist ein erheblicher operativer Aufwand erforderlich.

Zur Fixierung der Osteotomie sind auch Knochenklammern bekannt, deren Anwendung jedoch die Gefahr von Knochenabsplitterungen mit sich bringt.

Aus der AT 000 937 U ist ein Implantat zur gegenseitigen Fixierung zweier Knochenfragmente, insbesondere zur Behandlung einer Achsenfehlstellung eines Mittelfußknochens, z.B. Hallux valgus, bekannt geworden, das eine Spange mit zwei Schenkeln aufweist, die an ihren einen Enden miteinander verbunden sind und in diesem Bereich eine Öffnung begrenzen, durch die eine in einem der beiden Knochenfragmente einschraubbare Schraube hindurchgeführt ist, und die mit ihren anderen, freien Enden in den Markraum des anderen Knochenfragmentes einführbar und ausspreizbar sind. Nach erfolgter Osteotomie wird die Spange mit den freien Enden der beiden Schenkel in den Markraum des einen Knochenfragmentes nach proximal eingesetzt und verspreizt sich in diesem Markraum, worauf die Spange durch die durch die Öffnung hindurchgeführte Schraube am anderen Knochenfragment verankert wird.

Der intramedulläre Anteil dieses bekannten Implantates hat durch die Spreizkraft und den Reibschluß der Spangenschenkel sowie durch die intermittierende Kompression, die bei funktioneller Belastung auftritt, eine hohe Stabilität. Der extramedulläre Anteil ist hingegen nur mit einer Schraube im Knochen verankert, benötigt jedoch wegen des Drehmomentes, das beim Nachlassen der Kompression der Zugschraubenverankerung zwischen dem Implantat und dem Metatarsalköpfchen vermehrt entsteht, eine Zweipunkt-Abstützung.

Um die Fixierung dieses bekannten Implantats mit dem Knochenfragment, das mit dem Implantat über die Schraube verbunden ist, zu verbessern und stabil zu gestalten, ist es bekannt, zwischen der Spangenöffnung und dem Schraubenkopf ein von der Schraube durchsetzten Beilagteil mit im Knochenfragment verankerbaren Ansätzen vorzusehen (WO 00/06036). Da dieser Beilagteil als separates Element ausgebildet ist, treten bei der Implantation zusätzliche Schwierigkeiten auf, und es werden zusätzliche Instrumente benötigt.

Die Aufgabe der Erfindung besteht in der Schaffung eines Implantats, das leichter zu implantieren ist und das im implantierten Zustand eine größere Stabilität sicherstellt.

Die erfindungsgemäße Lösung besteht darin, daß das Implantat eine in die Spangenöse eingesetzte, daran befestigte Führungsbuchse für die Schraube aufweist, deren Innenfläche im wesentlichen der Außenfläche der Schraube im an den Schraubenkopf anschließenden Bereich entspricht und deren minimaler Innendurchmesser mindestens so groß ist, wie der größte Durchmesser des Schraubengewindes.

Es ist also kein Beilagteil mehr vorgesehen, sondern eine Führungsbuchse, die fest an der Spange befestigt ist. Dadurch wird die Implantation einfacher, und es sind keine zusätzlichen Werkzeuge erforderlich. Die Erfindung erschöpft sich aber nicht darin. Vielmehr hat die Führungsbuchse eine erhöhte Stabilität zur Folge, da die Schraube im implantierten Zustand im wesentlichen spielfrei in dieselbe eingesetzt ist, so daß die Winkelstabilität zwischen Spange und Schraube sichergestellt ist.

Die Innenfläche der Führungsbuchse könnte z.B. leicht konisch ausgebildet sind, wobei dann die Außenfläche der Schraube im an den Schraubenkopf anschließenden Bereich entsprechend ausgebildet ist, so daß die Schraube im wesentlichen spielfrei in der Führungsbuchse angeordnet werden kann. Bei einer besonders vorteilhaften Ausführungsform sind aber die Innenfläche der Führungsbuchse und die entsprechende Außenfläche der Schraube im wesentlichen zylindrisch.

Die Führungsbuchse ist bei einer vorteilhaften Ausführungsform dadurch an der Spangenöse befestigt, daß sie einen die Spangenöse wenigstens teilweise umgreifenden Randbereich aufweist. Sie kann in diesem Falle an der Spangenöse durch Umbiegen des Randbereiches dauerhaft befestigt werden. Dabei kann die Führungsbuchse eine die Spangenöse wenigstens teilweise aufnehmende ringförmige Vertiefung aufweisen.

Die Fläche, mit der das Implantat an dem Knochenfragment anliegt, das mit dem Implantat über die Schraube verbunden ist, muß gegenüber der Schenkelebene versetzt sein. Dies geschieht beim Stand der Technik dadurch, daß die die Spangenöse bildenden Enden der Schenkel im wesentlichen in einer Ebene liegen, die zur Schenkelebene, in der der übrige Teil der Schenkel liegt, parallel und versetzt ist. Dies kann bei dem erfindungsgemäßen Implantat ebenfalls vorgesehen sein, ist aber nicht erforderlich, da die Fläche, mit der das Implantat an dem Knochenfragment anliegt, welches mit der Schraube verbunden ist, aufgrund der axialen Länge der Führungshülse seitlich versetzt ist, wenn die Führungshülse sich von der Spangenöse zur Anlagefläche axial erstreckt. Auf jeden Fall kann aber die Abbiegung des Bereiches der Spangenöse geringer gestaltet werden, was den Vorteil hat, daß das Material hier beim Biegen nicht so stark beansprucht wird und deswegen die Gefahr sehr viel kleiner ist, daß es in diesem Bereich zu Materialbrüchen kommt. Die seitliche Versetzung der Spangenöse, die trotzdem vorgenommen werden kann, hat aber den Vorteil, daß in diesem Falle die Schraube nicht über die Schenkelebene vorsteht.

Vorteilhafterweise weist die Innenfläche der Führungsbuchse eine Länge von mindestens 3 mm in axialer Richtung auf.

Die Erfindung wird im folgenden anhand von vorteilhaften Ausführungsformen unter Bezugnahme auf die beigefügten Zeichnungen beschrieben. Es zeigen:
- Fig 1.: die Verwendung eines vorbekannten Implantats bei der Rekonstruktion eines Hallux valgus;
- Fig. 2: das Implantat des Standes der Technik in Draufsicht;
- Fig. 3: das Implantat des Standes der Technik in Seitenansicht;
- Fig. 4: eine Ausführungsform des erfindungsgemäßen Implantats in Draufsicht;
- Fig. 5: die Ausführungsform der Fig. 4 in Seitenansicht; und
- Fig. 6 bis 7: Querschnittsansichten entsprechend der Linie VI-VI von Fig. 5 durch verschiedene Ausführungsformen des erfindungsgemäßen Implantats.

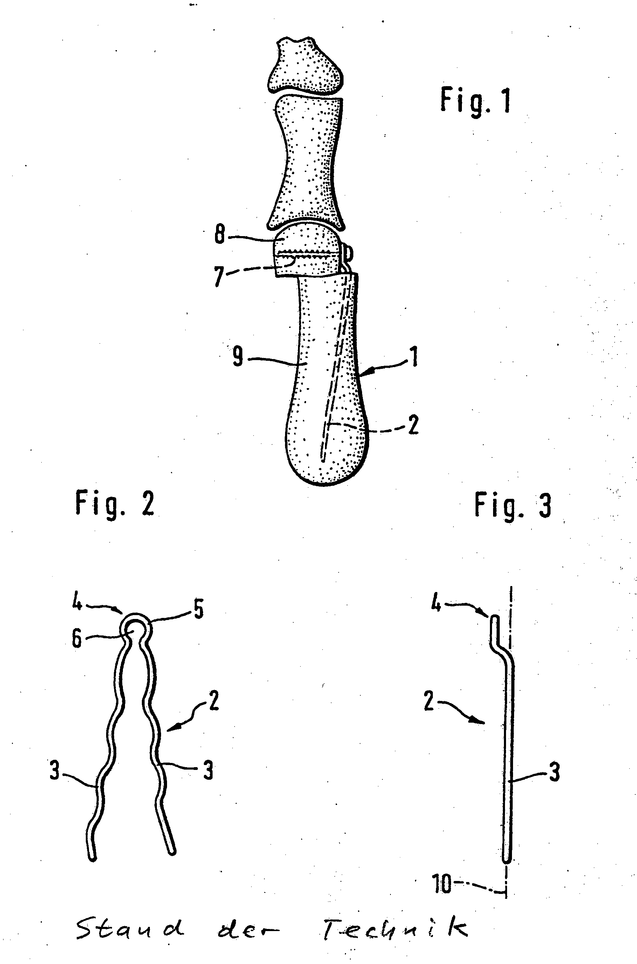

In Fig. 1 ist ein Mittelfußknochen 1 nach erfolgter Osteotomie und Reposition dargestellt. Zur Fixierung wird beim Stand der Technik ein Implantat verwendet, das eine in den Fig. 1 bis 3 dargestellte Spange 2 aufweist. Diese Spange weist zwei Schenkel 3 auf, deren eine Enden bei 4 miteinander verbunden sind und dort eine von einer Öse 5 gebildete Öffnung 6 begrenzen, durch die eine Kleinfragmentschraube 7 (siehe Fig. 1) hindurchführbar ist, die im Knochenfragment 8 in üblicher Weise verankert wird.

Die beiden Schenkel 3 der Spange 2 werden in den Markraum des Knochenfragments 9 eingeführt und nehmen dort ihre in Fig. 2 dargestellte ausgespreizte Lage ein, wobei die Spange 2 in diesem Markraum verankert wird. Die Verankerung wird durch eine vorzugsweise in der Schenkelebene verlaufende Wellung unterstützt.

Wie aus den Fig. 1 und 3 hervorgeht, sind die die Öffnung 6 begrenzenden, miteinander bei 4 verbundenen Enden der beiden Schenkel aus der vom übrigen Teil der Schenkel 3 aufgespannten Schenkelebene 10 herausgebogen und liegen in einer zu dieser Schenkelebene 10 parallelen Ebene. Dadurch wird die laterale Verschiebung der beiden Knochenfragmente 8, 9 berücksichtigt. Der Abstand zwischen diesen Ebenen ist dieser lateralen Verschiebung angepaßt.

Die Spange 2 des Standes der Technik kann aus einem biegeelastischen Stahldraht bestehen, wobei sich der Abstand der beiden Schenkel 3 im unbelasteten Zustand von der Verbindungsstelle 4 ausgehend in Richtung zu den freien Enden der Schenkel vergrößert. Beim Einführen der Schenkel 3 in den Markraum werden diese zusammengedrückt und verspreizen sich nach Beendigung des manuell auf die Schenkel ausgeübten Drucks im Markraum.

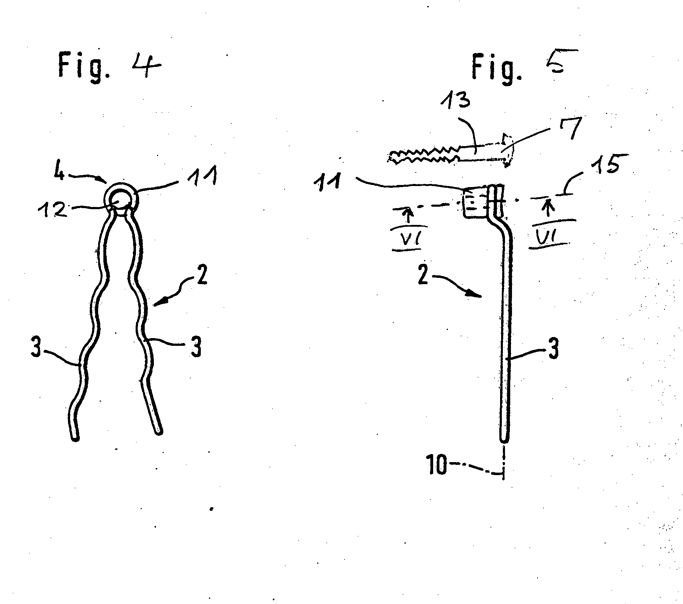

Obwohl beim Implantat des Standes der Technik der Fig. 1 bis 3 die Schraube 7 im implantierten Zustand die Öse 5 gegen das Knochenfragment 8 drückt, kann eine gewisse Winkelbewegung bei Belastung der Knochen nicht ausgeschlossen werden, was ganz offensichtlich zu ernsthaften Problemen führen kann. Dies kann erfindungsgemäß verhindert werden, indem die Schraube nicht einfach von der Öse 5 gehalten wird, sondern indem in die Öse 5 eine Führungsbuchse 11 eingesetzt wird, die in den Fig. 4 bis 7 gezeigt ist. Diese Führungsbuchse 11 weist eine zylindrische Durchgangsbohrung 12 auf, die dem zylindrischen Teil 13 in der Nähe des Kopfes der Schraube 7 entspricht. Dadurch ist eine Winkelbewegung der Schraube 7 in der Führungsbuchse 11 und damit gegenüber der Spange 2 verhindert. Wie dies in Fig. 5 gezeigt ist, ist dabei die Achse 15 der Bohrung der 12 der Führungsbuchse 11 gegenüber der Schenkelebene 10 in einem von 90° verschiedenen Winkel angeordnet, um so eine bessere Anpassung an die Kraftresultierende zu erzielen, insbesondere in einem Winkel von ungefähr 75°.

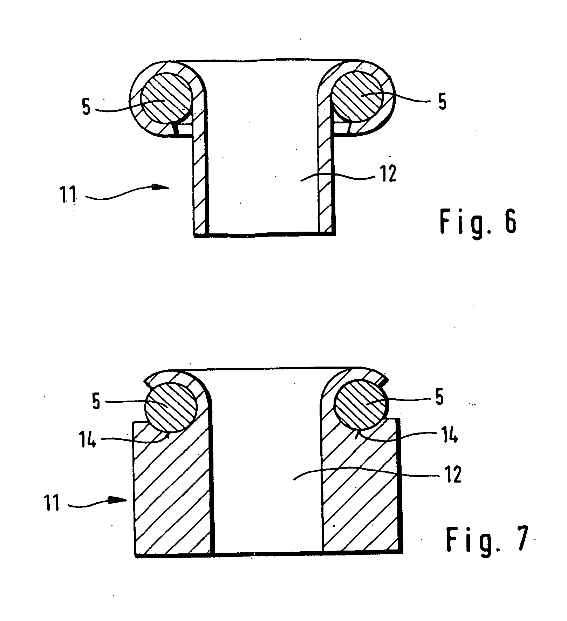

Wie dies in Fig. 6 gezeigt ist, weist die Führungsbuchse 11 einen Rand auf, der um die Öse 5 herumgebogen ist, um so die Führungsbuchse 11 zuverlässig festzuhalten. Bei der Ausführungsform der Fig. 7, die in etwa der Ausführungsform der Fig. 5 entspricht, weist die Führungsbuchse 11 einen größeren Außendurchmesser und Vertiefungen 14 auf, die die Öse 5 teilweise aufnehmen.

## Patentansprüche

1. Implantat (2) mit Schraube (7) zur gegenseitigen Fixierung zweier Knochenfragmente (8, 9), insbesondere zur Fixierung eines in der Achse korrigierten Köpfchens eines Mittelfußknochens, z.B. Hallux valgus, das eine Spange (2) mit zwei Schenkeln (3) aufweist, die an ihren einen Enden miteinander verbunden sind und in diesem Bereich eine Öse (5) mit einer Öffnung (6) bilden, durch die eine in einem (8) der beiden Knochenfragmente einschraubbare Schraube (7) hindurchgeführt ist, und die mit ihren anderen, freien Enden in das andere Knochenfragment (9) einführbar sind, **dadurch gekennzeichnet, dass** es eine in die Spangenöse (5) eingesetzte daran befestigte Führungsbuchse (11) für die Schraube (7) aufweist, deren Innenfläche im wesentlichen der Außenfläche der Schraube (7) im an den Schraubenkopf anschließenden Bereich entspricht und deren minimaler Innendurchmesser mindestens so groß ist wie der größte Durchmesser des Schraubengewindes, wobei die Schraube im wesentlichen spielfrei in der Führungsbuchse (11) gehalten wird.

2. Implantat nach Anspruch 1, **dadurch gekennzeichnet, dass** die Innenfläche der Führungsbuchse (11) im wesentlichen zylindrisch ist.

3. Implantat nach einem der Ansprüche 1 bis 2, **dadurch gekennzeichnet, dass** die Führungsbuchse (11) einen die Spangenöse (5) wenigstens teilweise umgreifenden Randbereich aufweist.

4. Implantat nach einem der Ansprüche 1 bis 3, **dadurch gekennzeichnet, dass** die Führungsbuchse (11) eine die Spangenöse (5) wenigstens teilweise aufnehmende ringförmige Vertiefung (14) aufweist.

5. Implantat nach einem der Ansprüche 1 bis 4, **dadurch gekennzeichnet, dass** die die Spangenöse (5) bildenden Enden der Schenkel (3) im wesentlichen in einer Ebene liegen, die zur Schenkelebene (10), in der der übrige Teil der Schenkel (3) liegt, parallel und versetzt ist.

6. Implantat nach einem der Ansprüche 1 bis 5, **dadurch gekennzeichnet, dass** die Innenfläche der Führungsbuchse (11) eine Länge von mindestens 3 mm in axialer Richtung aufweist.

7. Implantat nach einem der Ansprüche 1 bis 6, **dadurch gekennzeichnet, dass** die Achse (15) der Innenfläche der Führungsbuchse (11) einen von 90° verschiedenen Winkel mit der Schenkelebene (10) bildet.

## Claims

1. Implant (2) having a screw (7) for fixing two bone fragments (8, 9) to each other, in particular for fixing an axially corrected capitulum of a metatarsal bone, e.g. hallux valgus, which implant comprises a clasp (2) with two arms (3) which are connected to each other at one of their ends and in this area form an eyelet (5) with an opening (6) for passage of a screw (7) which can be screwed into one (8) of the two bone fragments, and which, with their other, free ends, can be introduced into the other bone fragment (9), **characterized in that** it has a guide bushing (11) for the screw (7), which guide bushing (11) is inserted into the clasp eyelet (5) and is secured thereon, its inner surface corresponding substantially to the outer surface of the screw (7) in the area adjoining the screw head, and its minimum internal diameter being at least as great as the maximum diameter of the screw thread, the screw being held in the guide bushing (11) substantially free of play.

2. Implant according to Claim 1, **characterized in that** the inner surface of the guide bushing (11) is substantially cylindrical.

3. Implant according to either of Claims 1 and 2, **characterized in that** the guide bushing (11) has an edge area engaging at least partially around the clasp eyelet (5).

4. Implant according to one of Claims 1 to 3, **characterized in that** the guide bushing (11) has an annular recess (14) at least partially receiving the clasp eyelet (5).

5. Implant according to one of Claims 1 to 4, **characterized in that** the ends of the arms (3) forming the clasp eyelet (5) lie substantially in a plane which is parallel and offset in relation to the plane (10) of the arms in which the remaining part of the arms (3) lies.

6. Implant according to one of Claims 1 to 5, **characterized in that** the inner surface of the guide bushing (11) has a length of at least 3 mm in the axial direction.

7. Implant according to one of Claims 1 to 6, **characterized in that** the axis (15) of the inner surface of the guide bushing (11) forms, with the plane (10) of the arms, an angle other than 90°.

## Revendications

1. Implant (2) à vis (7) pour la fixation réciproque de deux fragments osseux (8, 9), en particulier pour la fixation d'une tête à axe corrigé d'un os médian du pied, par exemple le hallux valgus, qui présente une agrafe (2) à deux branches (3) qui sont reliées entre elles par une de leurs extrémités et forment dans cette zone un oeillet (5) pourvu d'un orifice (6) à travers lequel passe une vis (7) pouvant être vissée dans un (8) des deux fragments osseux et qui peuvent être introduites dans l'autre fragment osseux (9) par leurs autres extrémités libres, **caractérisé en ce qu'**il présente un manchon de guidage (11) inséré dans l'oeillet (5) de l'agrafe et fixé dessus pour la vis (7) et dont la surface intérieure coïncide sensiblement avec la surface extérieure de la vis (7) dans la zone se raccordant à la tête de vis et dont le diamètre intérieur minimal est au moins aussi grand que le plus grand diamètre du filetage, la vis étant maintenue sensiblement sans jeu dans le manchon de guidage (11).

2. Implant selon la revendication 1, **caractérisé en ce que** la surface intérieure du manchon de guidage (11) est sensiblement cylindrique.

3. Implant selon l'une quelconque des revendications 1 à 2, **caractérisé en ce que** le manchon de guidage (11) présente une partie périphérique entourant au moins partiellement l'oeillet (5) de l'agrafe.

4. Implant selon l'une quelconque des revendications 1 à 3, **caractérisé en ce que** le manchon de guidage (11) présente un creux de forme annulaire (14) recevant au moins partiellement l'oeillet (5) de l'agrafe.

5. Implant selon l'une quelconque des revendications 1 à 4, **caractérisé en ce que** les extrémités formant l'oeillet (5) de l'agrafe des branches (3) se situent sensiblement dans un plan qui est parallèle et décalé par rapport à un plan (10) des branches dans lequel se trouve la partie restante des branches (3).

6. Implant selon l'une quelconque des revendications 1 à 5, **caractérisé en ce que** la surface intérieure du manchon de guidage (11) a une longueur d'au moins 3 mm dans le sens axial.

7. Implant selon l'une quelconque des revendications 1 à 6, **caractérisé en ce que** l'axe (15) de la surface intérieure du manchon de guidage (11) forme un angle différent de 90° avec le plan (10) des branches.
